(19) Europäisches Patentamt
European Patent Office
Office européen des brevets

(11) **EP 2 415 493 A1**

(12) **EUROPEAN PATENT APPLICATION**
published in accordance with Art. 153(4) EPC

(43) Date of publication:
08.02.2012 Bulletin 2012/06

(51) Int Cl.:
*A61M 5/32* (2006.01)

(21) Application number: 10761534.6

(22) Date of filing: 05.03.2010

(86) International application number:
PCT/JP2010/053681

(87) International publication number:
WO 2010/116832 (14.10.2010 Gazette 2010/41)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(30) Priority: 30.03.2009 JP 2009083417

(71) Applicant: Terumo Kabushiki Kaisha
Tokyo 151-0072 (JP)

(72) Inventors:
• KOIWAI Kazunori
Ashigarakami-gun
Kanagawa 259-0151 (JP)
• IWASE Yoichiro
Ashigarakami-gun
Kanagawa 259-0151 (JP)

(74) Representative: TBK
Bavariaring 4-6
80336 München (DE)

(54) **SYRINGE NEEDLE ASSEMBLY AND MEDICAMENT INJECTION DEVICE**

(57) An injection needle assembly includes a needle tube 2 having a needle tip capable of being stuck into a living body, a hub 3 holding the needle tube 2, a stabilization unit 6 arranged so as to cover the circumference of the needle tube 2 in a condition that the needle tip of the needle tube 2 protrudes and having an end face 6c that contacts the skin when causing the needle tube 2 to be stuck into the living body, and housing units 7 and 8. The housing units 7 and 8 are movable along the axial direction of the needle tube 2 from the first position where the needle tip of the needle tube 2 is exposed to the second position where the needle tip of the needle tube 2 is housed.

*FIG. 1*

**Description**

Technical Field

The present invention relates to a syringe needle assembly (injection needle assembly) and a medicament injection device (drug injection device) that are used to inject a drug into a skin upper layer part by sticking a needle tip from the skin surface.

Background Art

Recently, human infection of bird flu has been reported, and heavy damage by human pandemic of bird flu is worried about. So, pre-pandemic vaccine that has a high possibility of being effective against bird flu is being stockpiled worldwide. Also, to administer pre-pandemic vaccine to many people, a study is being carried out on expanding the production quantity of vaccine.

Skin is composed of three parts, an epidermis, a dermis, and a subcutaneous tissue. The epidermis is a layer of about 50-200 μm from the skin surface, and the dermis is a layer of about 1.5-3.5 mm continuing from the epidermis. Because flu vaccine is generally subcutaneously or intramuscularly administered, it is administered to a lower layer part of skin or a portion deeper than that.

On the other hand, it has been reported that by administering flu vaccine to an upper layer part of skin as a target site where many immunocompetent cells are present, even if the dosage is reduced, the same immunity acquisition ability as in subcutaneous administration or intramuscular administration can be obtained (Non-patent Document 1). Thus, by administering pre-pandemic vaccine to the skin upper layer part, the dosage can be reduced, so that it will result in that pre-pandemic vaccine can be administered to more people. Note that the skin upper layer part refers to the epidermis and dermis of skin.

As the method of administering a drug to the skin upper layer part, methods using a single-needle, a multi-needle, a patch, gas, etc. have been reported. And, if stability and reliability of administration and production cost are considered, as the method of administering a drug to the skin upper layer part, the method using a single needle is regarded most suitable. As the method of administering vaccine to the skin upper layer part using a single needle, a Mantoux method has been known from a long time ago. The Mantoux method is a method in which a needle, generally 26-27 G in size and having a short bevel needle tip, is inserted into skin about 2-5 mm, from an oblique direction of about 10-15° relative to the skin, to administer a drug of about 100 μl.

However, the Mantoux method is difficult in manipulation, and the rate of success is left in the skill of the doctor giving an injection. In particular, a child has a possibility of moving at the time of administration, so that it is difficult to administer flu vaccine with the Mantoux method. Accordingly, it is desired that a device is developed that can administer vaccine to the skin upper layer part in a simple and convenient manner.

Patent Document 1 describes an injection device for the skin upper layer part in which a limiter having a skin contacting surface is connected with a needle hub. The limiter described in Patent Document 1 is provided around a needle tube, and has a gap with the needle tube. By regulating the length (protrusion length) of the needle tube protruding from the surface of the limiter contacting the skin to 0.5-3.0 mm, the drug is administered in the skin.

Patent Document 1: Japanese Unexamined Patent Application Publication No. 2001-137343

Non-patent Document 1: R. T. Kenney et al. New England Journal of Medicine, 351, 2295-2301 (2004)

Disclosure of the Invention

Problems to be Solved by the Invention

However, in the injection device described in Patent Document 1, because the needle tip of the needle tube protrudes from the surface of the limiter contacting the skin, there has been a problem that after the drug has been administered or when discarding the injection device, the needle tip of the needle tube might be accidentally stuck into the user.

The present invention aims in view of the above-described problems to provide an injection needle assembly and a drug injection device that prevent the needle tip of the needle tube from being accidentally stuck into the user after the drug has been administered or when discarding the drug injection device.

Means for Solving the Problems

To solve the above-described problems and achieve the object of the present invention, an injection needle assembly of the present invention includes a needle tube having a needle tip capable of being stuck into a living body, and a hub holding the needle tube. The injection needle assembly further includes a stabilization unit arranged so as to cover the circumference of the needle tube in a condition that the needle tip of the needle tube protrudes. The stabilization unit has an end face that contacts a skin when causing the needle tube to be stuck into the living body. The injection needle assembly unit further includes a housing unit movable along an axial direction of the needle tube from a first position where the needle tip of the needle tube is exposed to a second position where the needle tip of the needle tube is housed.

A drug injection device of the present invention includes a needle tube having a needle tip capable of being stuck into a living body; a hub configured to hold the needle tube; and a syringe configured to be connected with the hub. The drug injection device further includes a stabilization unit arranged so as to cover the circumference of the needle tube in a condition that the needle tip of the needle tube protrudes, the stabilization unit having an end face that contacts a skin when causing the needle tube to be stuck into the living body; and a housing unit movable along an axial direction of the needle tube from a first position where the needle tip of the needle tube is exposed to a second position where the needle tip of the needle tube is housed.

Effects of the Invention

According to the injection needle assembly and the drug injection device of the present invention, a housing unit for housing the needle tip of the used needle tube is provided. As a result, it is possible to hold the injection needle assembly and the drug injection device after use in a safe condition, and it can be prevented that the needle tip of the needle tube is caused to be stuck in the user contrary to the user's intentions after administering the drug.

Brief Description of Drawings

Fig. 1 is a perspective view illustrating a first embodiment of an injection needle assembly of the present invention.
Fig. 2 is a cross-sectional view illustrating the first embodiment of the injection needle assembly of the present invention.
Fig. 3 is a perspective view illustrating the first embodiment of the injection needle assembly with a housing unit thereof removed.
Fig. 4 is a perspective view illustrating the housing unit of the injection needle assembly according to the first embodiment of the present invention.
Fig. 5 is a perspective view illustrating an outer housing unit of the injection needle assembly according to the first embodiment of the present invention, when viewed from the opposite side.
Fig. 6 is a perspective view illustrating the first embodiment of the injection needle assembly with the outer housing unit and the inner housing unit moved to a second position.
Fig. 7 is a cross-sectional view illustrating the first embodiment of the injection needle assembly with the outer housing unit and the inner housing unit moved to the second position.
Fig. 8 is a perspective view illustrating a second embodiment of the injection needle assembly of the present invention.
Fig. 9 is a perspective view illustrating the second embodiment of the injection needle assembly of the present invention with the outer housing unit moved to the second position.
Fig. 10 is a perspective view illustrating a third embodiment of the injection needle assembly of the present invention.
Fig. 11 is a cross-sectional view illustrating a third embodiment of the injection needle assembly of the present invention assembly before being used.
Fig. 12 is a perspective view illustrating the third embodiment of the injection needle assembly of the present invention with the inner housing unit moved to the second position.

Best Mode for Carrying out the Invention

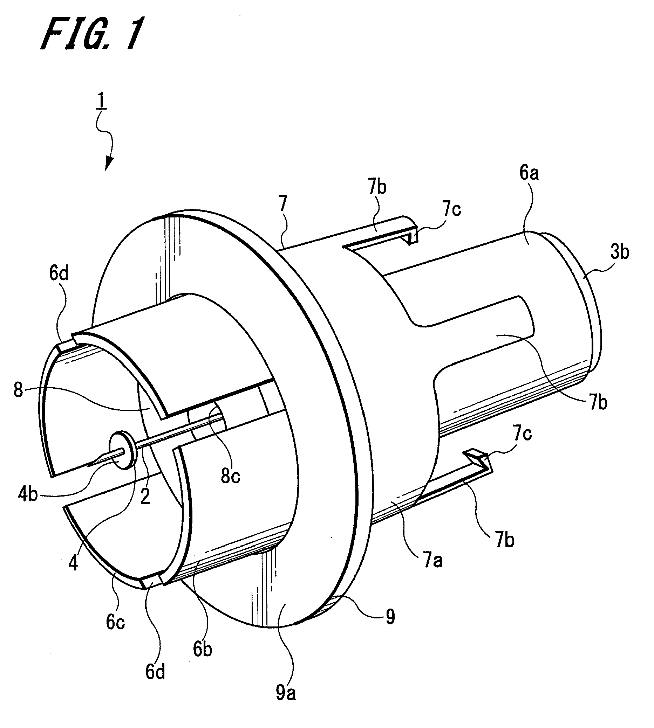

Below, description will be made, referring to Fig. 1 through Fig. 12, with respect to embodiments of an injection needle assembly and a drug injection device of the present invention. Note that the common parts in respective drawings are denoted by the same references numerals. The present invention is not limited to the following embodiments. The description will be made in the following order.

1. First embodiment

1.1. Exemplary constitutions of an injection needle assembly and a drug injection device
1.2. Method of using a drug injection device
2. Second embodiment
3. Third embodiment

1. First embodiment

1.1. Exemplary constitutions of an injection needle assembly and a drug injection device

First, referring to Fig. 1 through Fig. 5, description is made with respect to an injection needle assembly and a drug injection device according to the first embodiment of the present invention.

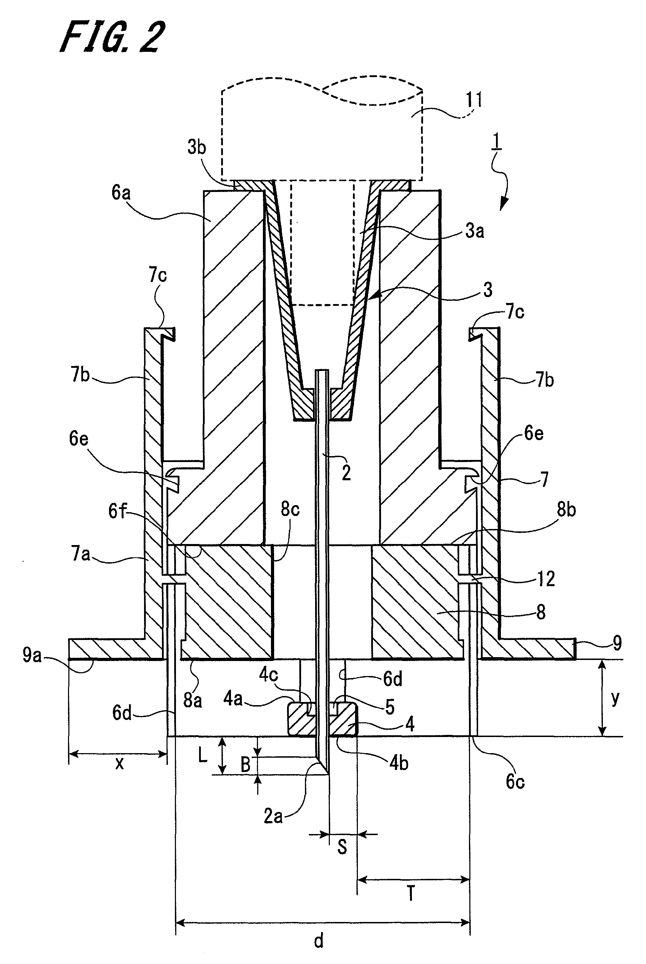

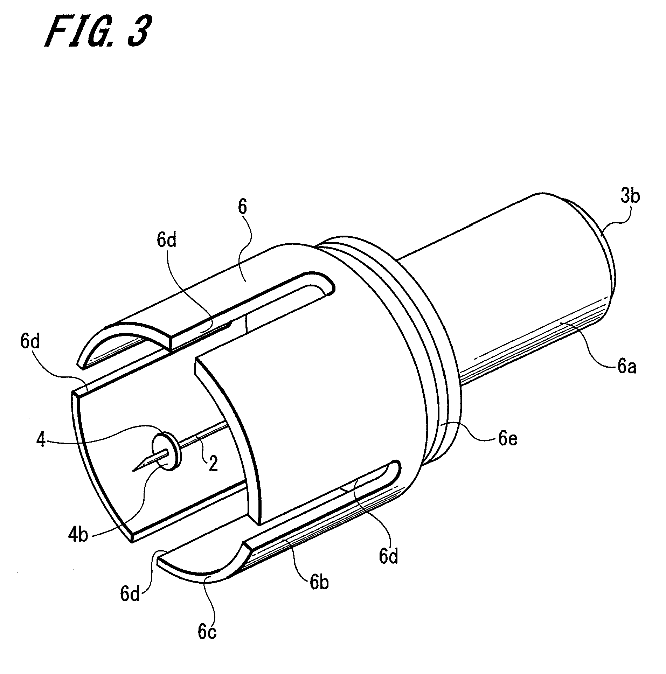

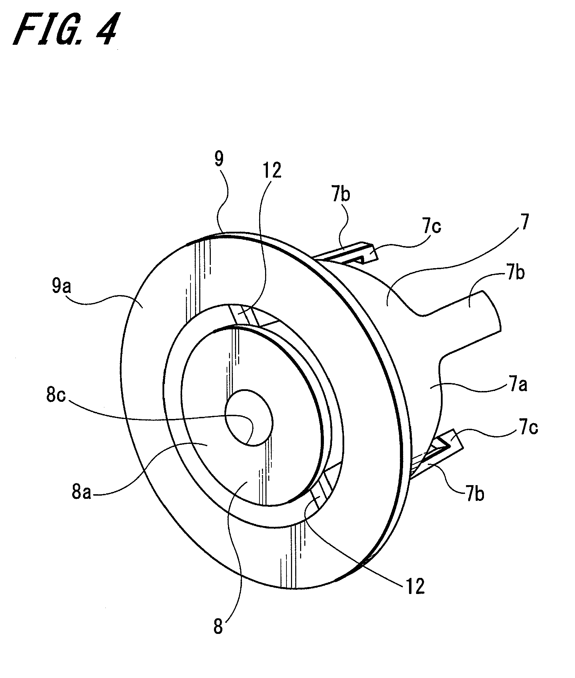

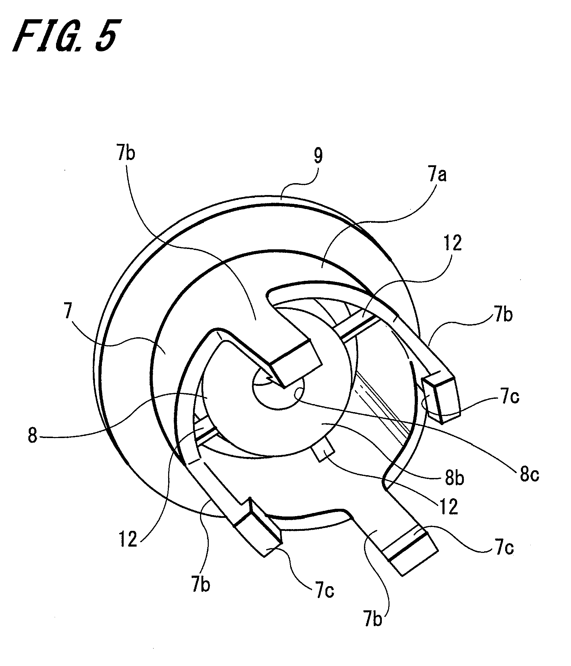

Fig. 1 is a perspective view illustrating the injection needle assembly of the present embodiment, Fig. 2 is a cross-sectional view illustrating the injection needle assembly of the present embodiment, Fig. 3 is a perspective view illustrating the injection needle assembly of the present embodiment with an outer housing unit thereof removed, and Fig. 4 and Fig. 5 are perspective views illustrating the outer housing unit of the present embodiment.
As illustrated in Fig. 1 and Fig. 2, an injection needle assembly 1 includes a hollow needle tube 2, a hub 3 that holds the needle tube 2, an adjustment unit 4 fixed to the needle tube 2, a stabilization unit 6, an outer housing unit 7 and an inner housing unit 8 that are movable along an axial direction of the needle tube 2, and an a guide unit 9. By connecting a syringe 11 to the hub 3, the injection needle assembly 1 is constituted as a drug injection device of the present invention (see Fig. 2).

[Needle tube]

For the needle tube 2, a needle tube with the size of 26-33G (outer diameter: 0. 2-0. 45 mm) by ISO standard for medical needle tubes (ISO9626: 1991/Amd.1:2001(E)), preferably the one with the size of 30-33G, is used. At the tip end part of the needle tube 2, a blade face 2a for making the needle tip acute-angled is formed. The blade face 2a may be of any length in the direction that the needle tube 2 extends (hereinafter, called "bevel length B", shown in Fig. 2) so long as it is 1.4 mm or below, which is the thinnest thickness of the later-described skin upper layer part in adults, and about 0. 5 mm or above, which is the bevel length when a short bevel has been formed in the needle tube of 33 G. That is, the bevel length B is preferably set in the range of 0.5-1.4 mm.
Further, the bevel length B is more preferably 0.9 mm or below, which is the thinnest thickness of the skin upper layer part in children, that is, in the range of 0.5-0.9 mm. Note that the short bevel refers to a blade face forming 18-25° with respect to the longitudinal direction of the needle, which is generally used in injection needles.
As the materials of the needle tube 2, for example, stainless steel can be given, but the materials are not limited to this, and aluminum, aluminum alloy, titanium, titanium alloy, and other metals may be used. Also, for the needle tube 2, a straight needle, and a tapered needle in which at least a portion thereof is tapered may be applied.
The tapered needle may be configured such that the base end portion that is fixed to the hub has a larger outer diameter compared with the tip end portion including the needle tip, and the middle portion thereof is tapered. Further, by providing the adjustment unit in the tapered portion, because of the existence of the slope of the tapered portion, the adjustment unit is prevented from being moved toward the base end. Thereby, even if the needle-protruding surface is strongly pressed against the skin, the length that the needle tip protrudes from the needle-protruding surface will never change, and the needle can be surely stuck to a predetermined depth of the skin.
The tube hole of the needle tube 2 is communicated with the hub 3. The hub 3 includes a hub body 3a that holds the needle tube 2, and a flange 3b that continues from the hub body 3a. The hub body 3a is tapered so as to be made smaller toward the tip end. At the tip end part of the hub body 3a, the base end part of the needle tube 2 is fixed. The flange 3b is provided at the base end part of the hub body 3a. The stabilization unit 6 is fixed to the flange 3b. The hub 3 may be in any form so long as it can be connected with the syringe 11.
The syringe 11 may be such a syringe that is to be filled with a drug when using the drug injection device, or a pre-filled syringe filled with a drug in advance. Also, as the drug that is filled in the syringe 11, vaccine may be cited, however, the drug may be those using macromolecular substances such as cytokine, etc., or hormones.

[Adjustment unit]

The adjustment unit 4 is formed in a cylindrical shape. The needle tube 2 penetrates through the adjustment unit 4, and the shaft center of the needle tube 2 and the shaft center of the adjustment unit 4 coincide with each other. The adjustment unit 4 is fixed in close contact with the circumferential surface of the needle tube 2. One end surface of the adjustment unit 4 forms a hub-opposing surface 4a opposing the hub 3 and the other end surface of the adjustment unit 4 forms a flat needle-protruding surface 4b from which the needle tip of the needle tube 2 protrudes.

In the hub-opposing surface 4a of the adjustment unit 4, a concave part 4c for adhesive agent is provided so as to surround the circumference of the needle tube 2. The adjustment unit 4 is fixed in close contact with the circumferential surface of the needle tube 2 by coating an adhesive agent 5 in the concave part 4c for adhesive agent in the state that the needle tube 2 has penetrated through. As the adhesive agent 5, cyanoacrylate, epoxy resin, light curing resin, etc. may be cited, however, the adhesive agent 5 may be those made of other resin.

The needle-protruding surface 4b of the adjustment unit 4 contacts the surface of skin and defines the depth the needle tube 2 is stuck, when the needle tube 2 is stuck into the skin upper layer part. That is, the depth that the needle tube 2 is stuck into the skin is determined by the protrusion length of the needle tube 2 from the needle-protruding surface 4b. Hereinafter, this length is called a protrusion length L.

The thickness of the skin upper layer part corresponds to the depth of the epidermis layer and the dermis layer from the skin surface, which is generally in the range of 0.5-3.0 mm. Therefore, the protrusion length L of the needle tube 2 can be set in the range of 0.5-3.0 mm.

Further, the thickness of the skin upper layer part of a deltoid muscle, which is a flu vaccine administration cite, was measured with respect to 19 children and 31 adults. Using an ultrasonic measurement device (NP60R-UBM high-resolution echo for small animals, NEPA GENE, CO., LTD.), the skin upper layer part having high ultrasonic reflectivity was imaged and the thickness thereof was measured. Here, because the measured values showed log-normal distribution, the range of MEAN $\pm$ 2SD was obtained by geometrical mean. The result showed 0.9-1.6 mm with respect to children, and with respect to adults, the results were 1.4-2.6 mm in the far part, 1.4-2.5 mm in the middle part, and 1.5-2.5 mm in the near part. From the above, the thickness of the skin upper layer part of the deltoid muscle was confirmed as 0.9 mm or above in the case of children and 1.4 mm or above in the case of adults. Consequently, in injection in the skin upper layer part of the deltoid muscle, the preferable protrusion length L of the needle tube 2 can be set in the range of 0.9-1.4 mm.

Further, by setting the protrusion length L of the needle tube 2 in this manner, it becomes possible to securely position the blade face 2a in the skin upper layer part. As a result, the medicinal solution discharging outlet opened in the blade face 2a can be positioned in the skin upper layer part, regardless of its position in the blade face 2a. Note that even when the medicinal solution discharging outlet is located in the skin upper layer part, if the needle tip is stuck deeper than the skin upper layer part, the medicinal solution escapes to the outside of the skin upper layer part (e.g., to the subcutaneous tissue) from between the side face of the needle tip end part and the incised skin. For this reason, it is important that the needle tip of the needle tube 2 and the blade face are securely located in the skin upper layer part.

Note that in the case of a needle tube larger than 26 G, it is difficult to make the bevel length B 1.0 mm or smaller. Accordingly, to set the protrusion length L of the needle tube 2 in the preferable range (0.9-1.4 mm), it is desirable to use a needle tube that is smaller than 26 G.

The needle-protruding surface 4b of the adjustment unit 4 is formed such that the distance S from the circumferential edge to the circumferential surface of the needle tube 2 is 1.4 mm or below, preferably in the range of 0.3-1.4 mm. This distance S from the circumferential edge of the needle-protruding surface 4b to the circumferential surface of the needle tube 2 is set considering that the needle protruding surface 4b depresses the skin around the needle tube 2 and pressure is given to a blister formed in the skin upper layer part. Thereby, when the needle-protruding surface 4b depresses the skin around the needle tube 2, it can be prevented that the administered drug leaks to the outside of the skin.

As the material of the adjustment unit 4, synthetic resin (plastic), such as polycarbonate, polypropylene, polyethylene, etc., may be used, or metals such as stainless, aluminum, etc. may be used.

In the present embodiment, the adjustment unit 4 has been fixed to the needle tube 2 using the adhesive agent 5, however, as the injection needle assembly of the present invention, the adjustment unit 4 may be fixed to the needle tube 2 by another method. For example, such methods are conceivable that the adjustment unit 4 is formed of metal and is fixed to the needle tube 2 by caulking, welding, etc., and that the adjustment unit 4 is formed of synthetic resin and is fixed to the needle tube 2 by welding and integral molding (especially, insert molding). Note that the adjustment unit 4 is not essential for solving the problems to be solved by the present invention, and the problems to be solved by the present invention can be solved without providing the adjustment unit 4 to the needle tube 2.

[Stabilization unit]

As illustrated in Fig. 3, the stabilization unit 6 has a shape that two circular cylinders that are different in diameter axially continue in series. The needle tube 2, the hub 3, and the adjustment unit 4 are arranged in the cylindrical hole of the stabilization unit 6. The stabilization unit 6 includes a fixing part 6a that is fixed to the hub 3 and a contact part 6b that covers the circumference of the needle tube 2 and the adjustment unit 4. One end portion of the fixing part 6a is fixed to the flange 3b of the hub 3 by a fixing method, such as an adhesive, etc. The hub body 3a of the hub 3 is housed in the cylindrical hole of the fixing part 6a. The contact part 6b is provided on the other end side of the fixing part 6a continuing circumferentially.

The contact part 6b is arranged so as to surround the circumference of the needle tube 2 and the adjustment unit 4 by fixing the fixing unit 6a to the hub 3. The inner diameter of the contact part 6b is set so as to be larger than the internal diameter of the fixing unit 6a. Returning to Fig. 2, an end face 6c on the one end side in the axial direction of the contact part 6b is located substantially on the same plane as the needle protruding surface 4b of the adjustment unit 4. And, the needle tube 2 is perpendicular to the plane formed by the end face 6c of the stabilization unit 6 and the needle protruding surface 4b of the adjustment unit 4.

Accordingly, when the needle tube 2 has been stuck into a living body, the needle protruding surface 4b of the adjustment unit 4 contacts the surface of skin, and the end face 6c of the stabilization unit 6 also contacts the surface of skin. Thereby, it is possible to support the needle tube 2 substantially perpendicular to the skin by the stabilization unit 6. As a result, it can be prevented that the needle tube 2 deviates, and it is enabled to cause the needle tube 2 to be stuck straight to the skin.

Note that the needle protruding surface 4b of the adjustment unit 4 does not need to be located on the same plane as the end face 6c of the stabilization unit 6. That is, even if the needle protruding surface 4b of the adjustment unit 4 is located on the other end side (on the fixing part 6a side) in the axial direction of the contact part 6b relative to the end face 6c, the object of the present invention can be attained. If a bulge of skin when the stabilization unit 6 is depressed to the skin is considered, it is preferable to set the distance in the axial direction of the contact part 6b between the planes formed by the needle protruding surface 4b of the adjustment unit 4 and the end face 6c, respectively, to 1.3 mm or below.

The inner diameter d of the contact part 6b of the stabilization unit 6 is set at a value equivalent to the diameter of a blister which is formed in the skin or larger than that. Specifically, the inner diameter d is set such that the distance T from the internal wall surface of the contact part 6b to the outer circumferential surface of the adjustment unit 4 is in the range of 4-15 mm. Thereby, it is possible to prevent that pressure is applied from the internal wall of the stabilization unit 6 to a blister and formation of the blister is thereby disturbed by the stabilization unit 6.

There is no upper limit for the distance T from the internal wall surface of the stabilization unit 6 to the outer circumferential surface of the adjustment unit 4 so long as it is 4 mm or above. However, if the distance T is increased, the outer diameter of the stabilization unit 6 and the outer diameter of the contact part 6b increase. If the outer diameter of the contact part 6b is increased, when sticking the needle tube 2 to a thin arm as in a child, it becomes difficult to cause the end face 6c of the contact unit 6b to contact the skin. Accordingly, when the thinness of children's arms is considered, it is preferable to specify the distance T from the internal wall surface of the stabilization unit 6 (contact part 6b) to the outer circumferential surface of the adjustment unit 4 to 15 mm at the maximum.

Also, if the distance S from the circumferential edge of the needle protruding surface 4b of the adjustment unit 4 to the circumferential surface of the needle tube 2 is 0.3 mm or above, the needle tube 2 will never penetrate the skin. Accordingly, if the distance T (4 mm or above) from the internal wall surface of the contact part 6b to the outer circumferential surface of the adjustment unit 4 and the diameter (about 0.3 mm) of the needle tube 2 are considered, the internal diameter d of the contact part 6b can be set to 9 mm or above.

Further, in the contact part 6b, four sliding grooves 6d are formed by making cuts in the axial direction from the end face 6c. The four sliding grooves 6d are arranged in the contact part 6b substantially at equal intervals. Also, an engaging groove 6e is provided in the outer circumferential surface on the other end side of the contact part 6b. The engaging groove 6e is formed continuing along the circumferential direction of the contact part 6b. Also, the outer housing unit 7 is arranged so as to cover the circumference of the stabilization unit 7.

[Outer housing unit and inner housing unit]

As illustrated in Fig. 4 and Fig. 5, the outer housing unit 7 includes a covering part 7a to house the needle tube 2 after use, and four leg parts 7b. The covering part 7a is formed substantially in a cylindrical shape, and the internal diameter thereof is set slightly larger than the outer diameter of the contact part 6b of the stabilization unit 6. The four leg parts 7b are arranged at substantially equiangular intervals at the other end in the axial direction of the covering part 7a and protrude in the axial direction of the covering part 7a. At end parts of the four leg parts 7b, engaging claws 7c to be fixed to the engaging groove 6e of the stabilization unit 6 (see Fig. 3) are provided, respectively. That is, a fixing mechanism is constituted by the engaging groove 6e of the stabilization unit 6 and the engaging claws 7c of the outer housing unit 7.

Further, the inner housing unit 8 is arranged in the tube hole of the covering part 7a of the outer housing unit 7. As illustrated in Fig. 2, the inner housing unit 8 is arranged to cover the circumference of the needle tube 2 between the contact part 6b of the stabilization unit 6 and the needle tube 2. The inner housing unit 8 is formed in a substantially cylindrical shape, and includes one end face 8a in a substantially circular shape at one end in the axial direction thereof, and other end face 8b that opposes the one end face 8a. An insertion hole 8c connecting substantially centers of the one end face 8a and the other end face 8b is formed. The needle tube 2 and the adjustment unit 4 are inserted in the insertion hole 8c. Further, the inner housing unit 8 is formed integrally with the outer housing unit 7 through four connection pins 12.

The four connection pins 12 are arranged at substantially equal intervals at the internal wall of the outer housing unit 7, and are inserted in the four sliding grooves 6d of the stabilization unit 6. The four connection pins 12 are slidable along the four sliding grooves 6d of the stabilization unit 6. The four connection pins 12 slide along the four sliding grooves 6d, and thereby the outer housing unit 7 and the inner housing unit 8 are made to be slidable along the axial direction of the needle tube 2 and the stabilization unit 6.

Returning to Fig. 1 and Fig. 2, in a state before the drug is administered in the human body, the outer housing unit 7 and the inner housing unit 8 are arranged in positions where the other end face 8b of the inner housing unit 8 and the internal wall surface 6f of the stabilization unit 6 contact each other. Herein below, this position is referred to as "a first position". When the outer housing unit 7 and the inner housing unit 8 are arranged in the first position, the needle tip of the needle tube 2 protrudes from the outer housing unit 7 and the inner housing unit 8 and is exposed. Therefore, when the outer housing unit 7 and the inner housing unit 8 are arranged in the first position, the needle tip of the needle tube 2 can be stuck to the living body.

Further, a guide part 9 is integrally formed in the outer circumferential surface of the covering part 7a of the outer housing unit 7. The guide part 9 is provided at the one end side in the axial direction of the covering part 7a continuously along the circumferential direction of the outer circumferential surface of the covering part 7a. The guide part 9 is formed as a ring-like flange protruding from the outer circumferential surface of the outer housing unit 7 in the outward radial direction, substantially perpendicularly. The guide part 9 includes a contact surface 9a that contacts the skin. The contact surface 9a is a flat surface that is substantially parallel to the end face 6c of the stabilization unit 6.

By causing the stabilization unit 6 to be pressed to skin until the contact surface 9a of the guide part 9 contacts the skin, it is possible to ensure that the force the stabilization unit 6 and the needle tube 2 press the skin is always at a predetermined value or above. Thereby, the part of the needle tube 2 protruding from the needle protruding surface 4b (corresponding to the protrusion length L) is surely stuck in the skin. And, the distance from the contact surface 9a of the guide part 9 to the end face 6c of the stabilization unit 6 in a state that the outer housing unit 7 is arranged in the first position is set such that the needle tube 2 and the stabilization unit 6 are pressed to skin with an appropriate pressing force and thereby the part of the needle tube 2 protruding from the needle protruding surface 4b can be surely stuck in the skin. Hereinafter, the length of this distance is referred to as "a guide part height y".

Note that the appropriate pressing force of the needle tube 2 and the stabilization unit 6 for causing the part of the needle tube 2 protruding from the needle protruding surface 4b to be surely stuck in the skin is, for example, 0.5-20 N. As a result, effects are obtained that the guide part 9 can ensure that the pressing force applied by the needle tube 2 and the stabilization unit 6 to the skin is always at a predetermined value or above by being caused to contact the skin and the needle tip and blade face 2a of the needle tube 2 can be securely positioned in the skin upper layer part, thereby giving the user a sense of safety.

As shown in Fig. 2, specifically, when the internal diameter d of the stabilization unit 6 is within the range of 12-14 mm, the guide part height y is in the range calculated according to the following expression 1 based on a length x from the protruding end face of the guide part 9 to the outer circumferential surface of the stabilization unit 6 (below, called a guide part length x).


```
Expression 1:

1.0 Ln(x) + 1.2 ‹ y ‹ 3.1 Ln(x) + 3.2
```


Note that when the internal diameter d of the stabilization unit 6 is 11 mm, the guide part height y is set, for example when the guide part length x is 0.5 mm, in the range of 0.75-2.6 mm.

As the material for the stabilization unit 6, the outer housing unit 7, and the inner housing unit 8, as in the adjustment unit 4, synthetic resin (plastic), such as polycarbonate, polypropylene, polyethylene, etc., may be used, or metals such as stainless steel, aluminum, etc. may be used.

The shape of the stabilization unit 6 is not limited to the circular cylinder, and the stabilization unit 6 may be formed, for example, in a rectangular cylinder, such as a square cylinder, a hexagonal cylinder, etc. , having a cylindrical hole in the center. Also, the shapes of the outer housing unit 7 and the inner housing unit 8 are set correspondingly to the shape of the stabilization unit 6. Further, description has been made with respect to the case that four pieces of the sliding grooves 6d and the connection pins 12 are formed, respectively, however, the numbers of the sliding grooves 6d and the connection pins 12 may be three or below, or five or more, respectively. Also, description has been made for the case that the outer housing unit 7 and the inner housing unit 8 are integrally formed, however, the outer housing unit 7 and the inner housing unit 8 may be separate members. In addition, description has been made with respect to the case that the engaging groove 6e that constitutes the fixing mechanism is continuously formed along the circumferential direction of the contact part 6b, however, a plurality of concave parts to be engaged with the engaging claws 7c, respectively, may be formed in the outer circumferential surface of the contact part 6b.

Further, description has been made with respect to the case that the stabilization unit 6 is fixed to the hub 3, however, the stabilization unit 6 may be fixed to the syringe 11 constituting a drug injection device. Also, in the present example, the stabilization unit 6 is fixed to the hub 3 using an adhesive agent, however, the stabilization unit 6 may be fixed to the hub 3 by another method. For example, as the method for forming the stabilization unit 6 with metal and fixing the stabilization unit 6 to the hub 3, caulking, etc. may be cited. Also, as the method for forming the stabilization unit 6 with synthetic resin and fixing the stabilization unit 6 to the hub 3, welding and integral molding (in particular, insert molding including the needle tube 2) may be cited.

1.2. Method of using a drug injection device

Next, description will be made with respect to a method of using a drug injection device to which the injection needle assembly 1 has been applied, referring to Fig. 1, Fig. 2, Fig. 6, and Fig. 7.

First, the end face 6c of the stabilization unit 6 is caused to face the skin. Thereby, the needle tip of the needle tube 2 faces the skin to which the needle tip is stuck. Note that as illustrated in Fig. 1 and Fig. 2, the outer housing unit 7 and the inner housing unit 8 are arranged in the first position, respectively, and the needle tip of the needle tube 2 is exposed. Next, the injection needle assembly 1 is moved substantially perpendicularly to the skin, the needle tube 2 is stuck into the skin, and the end face 6c of the stabilization unit 6 is pressed to the skin. Here, the needle protruding surface 4b of the adjustment unit 4 and the end face 6c of the stabilization unit 6 are on the same plane. Thereby, the needle protruding surface 4b of the adjustment unit 4 can contact the skin and deform the skin so as to be flat, and the needle tube 2 can be stuck into the skin by the protrusion length L.

Next, the stabilization unit 6 is pressed to skin until the contact surface 9a of the guide part 9 contacts the skin. Here, the guide part height y is set such that the needle tube 2 and the stabilization unit 6 are pressed to skin with an appropriate pressing force and the part of the needle tube 2 protruding from the needle protruding surface 4b is surely stuck into the skin. Therefore, the force to press the skin with the stabilization unit 6 becomes a predetermined value. Accordingly, it is possible to ensure that the pressing force of the stabilization unit 6 to the skin is at a predetermined value by causing the guide part 9 to contact the skin and press the stabilization unit 6 to the skin with a suitable pressing force, and the needle tip and the blade face 2a of the needle tube 2 can be surely positioned in the skin upper layer part. Thus, since the guide part 9 serves as a mark for ensuring that the pressing force of the stabilization unit 6 is at a predetermined value by being caused to contact the skin, it is possible to reliably position the needle tip of the needle tube 2 in the skin upper layer part, administer the drug in the skin upper layer part, and improve the sense of safety of the user.

Also, by causing the stabilization unit 6 to contact the skin, the needle tube 2 is stabilized, and the needle tube 2 can be stuck straight to the skin. Accordingly, deviation caused in the needle tube 2 can be prevented, and stable administration of drug can be carried out. Also, with the needle having a very short protrusion length of about 0.5 mm for example, even if the needle tip is caused to contact the skin, there is a case that the needle tip is not stuck into the skin. However, if the stabilization unit 6 is pressed to the skin and the skin is depressed in a perpendicular direction, the skin on the inner side of the stabilization unit 6 is pulled and put in a condition that a tensional force has been applied to the skin. Therefore, the skin becomes hard to flee from the needle tip of the needle tube 2, so that the stabilization unit 6 has an effect that it becomes easier for the needle tip to be stuck into the skin.

Further, the protrusion length L being set in the range of 0.5-3.0 mm, the needle tip and the blade face 2a of the needle tube 2 are reliably positioned in the skin upper layer part. Thereafter, a drug is injected into the skin upper layer part with the syringe 11 connected with the hub 3.

The adjustment unit 4 of the injection needle assembly 1 is fixed in close contact with the circumference of the needle tube 2, and there is no gap between the adjustment part 4 and the part of the needle tube 2 penetrating the adjustment part 4. Therefore, if the needle protruding surface 4b of the adjustment unit 4 is caused to contact the skin, the skin around the needle tube 2 can be deformed to be flattened. As a result, the needle tube 2 can be stuck in the skin by the protrusion length L, and the needle tip of the needle tube 2 can be reliably positioned in the skin upper layer part.

Further, because the needle protruding surface 4b of the adjustment unit 4 and the internal diameter d of the stabilization unit 6 are set in suitable sizes, it becomes possible to cause the injected drug not to leak to the outside of the body, and the drug can be reliably administered in the skin upper layer part.

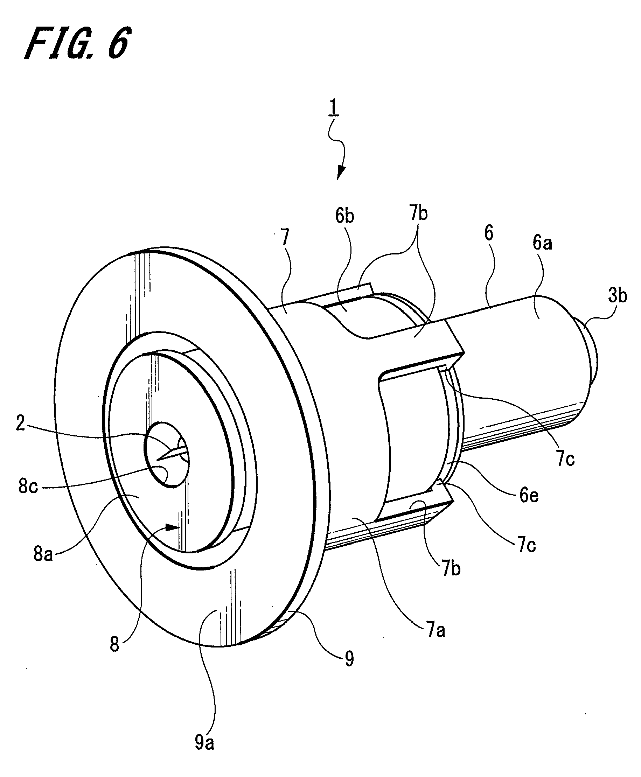

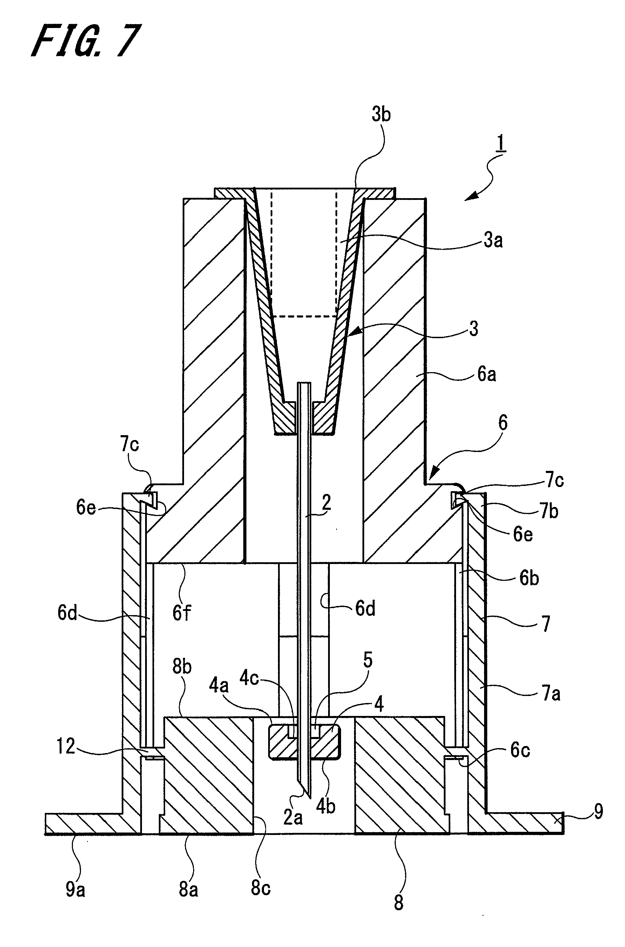

Next, as illustrated in Fig. 6 and Fig. 7, the outer housing unit 7 and the inner housing unit 8 of the injection needle assembly 1 after administering the drug are caused to slide along the axial direction of the needle tube 2, and the engaging claws 7c are fixed in the engaging groove 6e. Below, the position of the outer housing unit 7 and the inner housing unit 8 at this time is referred to as "a second position". Note that because a clicking feeling and a clicking sound are generated when fixing the engaging claws 7c in the engaging groove 6e, the user can confirm that the outer housing unit 7 and the inner housing unit 8 have been surely moved to the second position. Here, the end part of the covering part 7a and the one end face 8a of the inner housing unit 8 are located on the distal end side beyond the needle tip of the needle tube 2. That is, the needle tube 2 is housed in the covering part 7a of the outer housing unit 7 and the insertion hole 8c of the inner housing unit 8. As a result, it becomes possible to prevent that the needle tip of the needle tube 2 is stuck in the living body contrary to user's intents.

Here, the inner housing unit 8 moves integrally with the outer housing unit 7, and is arranged so as to close an opening of the covering part 7a of the outer housing unit 7. That is, the inner housing unit 8 is arranged so as to fill the space between the outer housing unit 7 and the needle tube 2, and thereby it is possible to prevent that the finger tip, etc. of the user enters into the cylinder hole of the stabilization unit 6 from the opening of the covering part 7a. Thereby, the needle tube 2 of the injection needle assembly 1 after use can be housed in a safer condition.

Also, by fixing the engaging claws 7c in the engaging groove 6e, it is prevented that the outer housing unit 7 and the inner housing unit 8 move from the second position to the first position. Therefore, there is no fear that the needle tube 2 housed in the outer housing unit 7 and the inner housing unit 8 protrudes from the outer housing unit 7 and the inner housing unit 8 again and is exposed. Thereby, it is possible to keep the injection needle assembly 1 after use always in a safe condition, and the user can carry out disposal, etc. of the used injection needle assembly 1 at ease.

2. Second embodiment

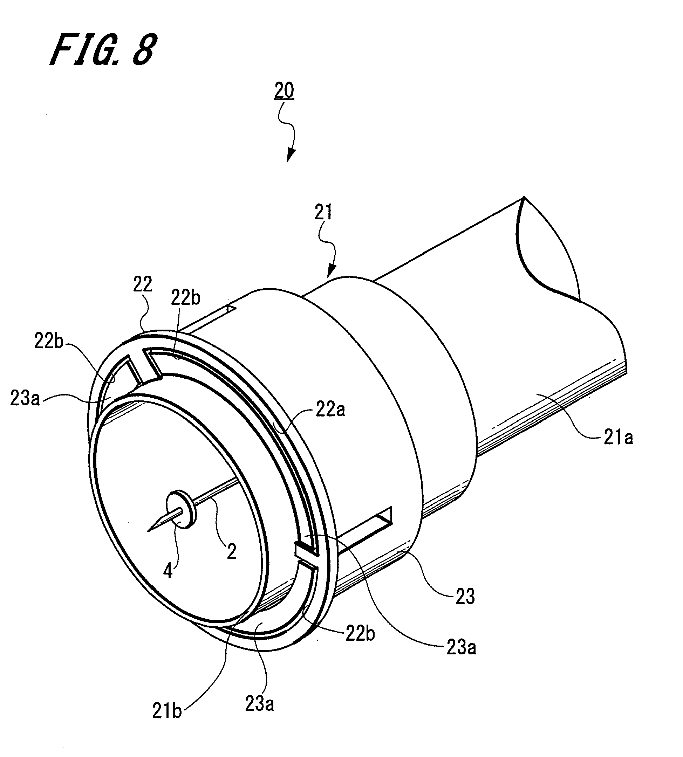

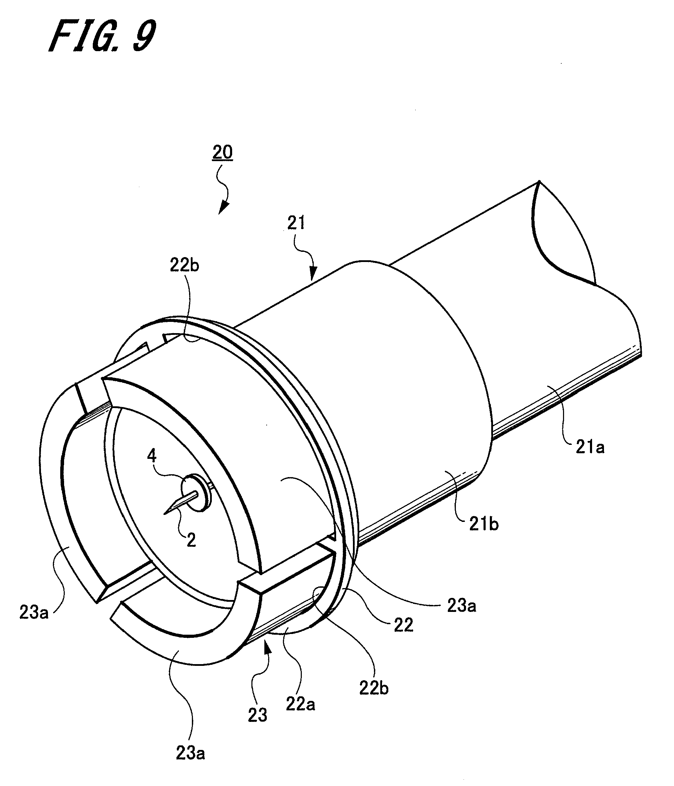

Next, referring to Fig. 8 and Fig. 9, description will be made with respect to an injection needle assembly according to the second embodiment of the present invention.
Fig. 8 and Fig. 9 are perspective views showing the injection needle assembly according to the second embodiment of the present invention.

An injection needle assembly 20 according to the second embodiment is constituted by removing the inner housing unit 8 of the injection needle assembly 1 according to the first embodiment and providing a guide part on a stabilization unit. Therefore, here, description will be made with respect to a stabilization unit, a guide part, and an outer housing unit, and the common parts with the injection needle assembly 1 are denoted with the same reference numerals and overlapped part of description will be omitted.

Note that the injection needle assembly 20 according to the second embodiment constitutes a drug injection device of the present invention by connecting a syringe to the hub 3 as in the above-described first embodiment.

As illustrated in Fig. 8, a stabilization unit 21 has a shape that two circular cylinders different in diameter continue in series in the axial direction. The needle tube 2, the hub 3, and the adjustment unit 4 are arranged in the cylindrical hole of the stabilization unit 21. The stabilization unit 21 includes a fixing part 21a, which is fixed to the hub 3, and a contact part 21b covering the circumference of the needle tube 2 and the adjustment unit 4. Further, although not shown in figure, an engaging groove or engaging concave part which constitutes a fixing mechanism is provided in the stabilization unit 21.

A guide part 22 is integrally molded in the outer circumferential surface of the contact part 21b of the stabilization unit 21. The guide part 22 is provided continuing along the circumferential direction of the outer circumferential surface of the contact part 21b of the stabilization unit 21. The guide part 22 is formed as a flange in a ring-like shape protruding from the outer circumferential surface of the stabilization unit 21 substantially perpendicularly in the outward radial direction. The guide part 22 includes a contact surface 22a which contacts the skin.

Further, three opening parts 22b are provided in the guide part 22 at substantially equiangular intervals. The three opening parts 22b are opened in the outward radial direction from the outer circumferential surface of the contact part 21b. An outer housing unit 23 is slidably arranged so as to cover the outer circumference of the stabilization unit 21.

The outer housing unit 23 is formed in a substantially circular cylinder shape, and the internal diameter thereof is set slightly larger than the outer diameter of the contact part 21b of the stabilization unit 21. The outer housing unit 23 is cutout in three places at substantially equal intervals in the axial direction, and thereby three covering parts 23a are formed. These three covering parts 23a are slidably inserted in the three opening parts 22b of the guide part 22. Thereby, the outer housing unit 23 is supported slidably along the axial direction of the stabilization unit 21 and the needle tube 2.

Also, although not shown in figure, protrusions that constitute a fixing mechanism are provided in the internal wall of the outer housing unit 23. The protrusions are engaged with the engaging groove or engaging concave part provided in the stabilization unit 21. By causing the protrusions and the engaging groove or engaging concave part to be engaged with each other, it is prevented that the outer housing unit 23 moves from the second position where the needle tube 2 is housed to the first position where the needle tube 2 is exposed.

As illustrated in Fig. 8, before use, the outer housing unit 23 is arranged in the position, which is the first position, where end faces of the three covering part 23a are substantially in the same plane with the contact surface 22a of the guide part 22. Then, as illustrated in Fig. 9, after administering the drug, the outer housing unit 23 is slid to the second position to cover the outer circumference of the needle tube 2 with the covering parts 23a. Thereby, the needle tube 2 can be housed in the outer housing unit 23, and the injection needle assembly 20 after use can be put in a safe condition.

Note that in the injection needle assembly 20 according to the second embodiment also, as in the injection needle assembly 1 according to the above-described first embodiment, an inner housing unit, which is slidably supported between the stabilization unit 21 and the needle tube 2, may be provided.

The constitution of other parts of the injection needle assembly 20 is similar to that of the injection needle assembly 1 according to the first embodiment, so that description thereof is omitted. With the injection needle assembly 20 having such constitution also, it is possible to obtain functions and effects similar to those of the injection needle assembly 1 according to the above-described first embodiment.


3. Third embodiment

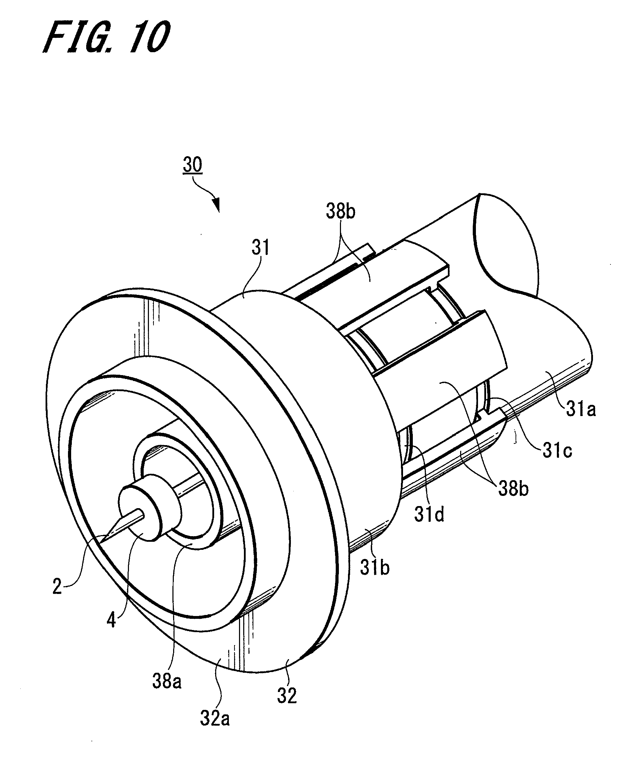

Next, referring to Fig. 10, Fig. 11, and Fig. 12, description will be made with respect to an injection needle assembly according to the third embodiment of the present invention.

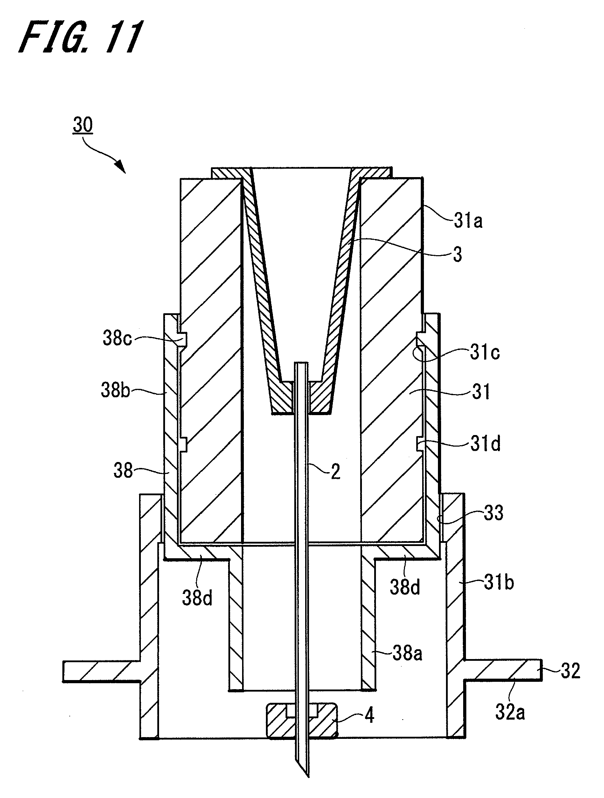

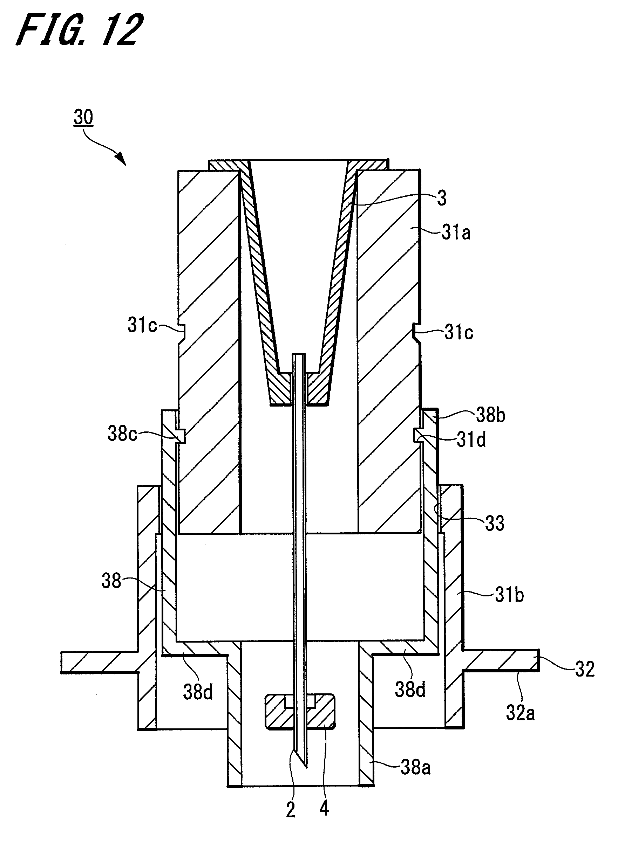

Fig. 10 is a perspective view illustrating the injection needle assembly according to the third embodiment of the present invention, and Fig. 11 and Fig. 12 are cross-sectional views illustrating the injection needle assembly according to the third embodiment of the present invention.

As illustrated in Fig. 10 and Fig. 11, an injection needle assembly 30 according to the third embodiment is constituted by removing the outer housing unit from the injection needle assembly 1. Therefore, here, description will be made with respect to a stabilization unit, a guide part, and an inner housing unit, and the common parts with the injection needle assembly 1 are denoted with the same reference numerals and overlapped part of description will be omitted.

Note that the injection needle assembly 30 according to the third embodiment constitutes a drug injection device of the present invention by connecting a syringe to the hub 3 as in the above-described first embodiment.

As illustrated in Fig. 10, a stabilization unit 31 has a shape that two circular cylinders different in diameter continue in series in the axial direction. The needle tube 2, the hub 3, and the adjustment unit 4 are arranged in the cylindrical hole of the stabilization unit 31. The stabilization unit 31 includes a fixing part 31a, which is fixed to the hub 3, and a contact part 31b covering the circumference of the needle tube 2 and the adjustment unit 4.

Further, a first engaging groove 31c and a second engaging groove 31d are provided in the outer circumferential surface of the fixing part 31a. The second engaging groove 31d is arranged closer to the contact part 31b of the fixing part 31a than the first engaging groove 31c.

A guide part 32 is integrally formed in the outer circumferential surface of the contact part 31b of the stabilization unit 31. The guide part 32 is continuously provided along the circumferential direction of the outer circumferential surface of the contact part 31b of the stabilization unit 31. The guide part 32 is formed as a flange in a ring-like shape protruding from the outer circumferential surface of the stabilization unit 31 substantially perpendicularly in the outward radial direction. The guide part 32 includes a contact surface 32a which contacts the skin.

Further, opening portions 33 are provided at substantially equiangular intervals at places where the contact part 31b and the fixing part 31a connect with each other. Leg parts 38b of an inner housing unit 38 described later movably penetrate through the opening portions 33.

The inner housing unit 38 includes a covering part 38a which houses the needle tube 2 after use, and a plurality of leg parts 38b provided continuously from the covering part 38a. The covering part 38a is formed in a substantially circular cylinder shape. The diameter of the covering part 38a is set smaller than the internal diameter of the contact part 31b. A connection part 38d is provided from one end in the axial direction of the covering part 38a. The connection part 38d protrudes from the outer circumferential surface of the covering part 38a substantially perpendicularly in the outward radial direction. The plurality of leg parts 38b are arranged at substantially equiangular intervals from the circumferential edge of the connection part 38d, and protrude in the opposite direction of the covering part 38a. Engaging claws 38c, which engage with the first engaging groove 31c and the second engaging groove 31d of the stabilization unit 31, are provided in end portions of the leg parts 38b, respectively.

As illustrated in Fig. 11, in a condition before use, the inner housing unit 38 is arranged such that the end face of the covering part 38a is positioned on the side of the fixing part 31a of the stabilization unit 31 than the needle tip of the needle tube 2. That is, the inner housing unit 38 is arranged in the first position where the needle tip of the needle tube 2 is exposed. At this time, the engaging claws of the leg parts 38b are engaged with the first engaging groove 31c of the stabilization unit 31.

Then, as illustrated in Fig. 12, after administering the drug, the inner housing unit 38 is moved to the second position by holding the leg parts 38b of the inner housing unit 38 to thereby cover the circumference of the needle tube 2 with the covering part 38a. Thereby, the needle tube 2 can be housed in the inner housing unit 38, and the injection needle assembly 30 after use can be put in a safe condition. At this time, the engaging claws 38c of the leg part 38b are fixed in the second engaging groove 31d of the stabilization unit 31. The second engaging groove 31d of the stabilization unit 31 and the engaging claws 38c of the inner housing unit 38 constitute a fixing mechanism.

The constitution of other parts of the injection needle assembly 30 is similar to that of the injection needle assembly 1 according to the first embodiment, so that description thereof is omitted. With the injection needle assembly 30 having the constitution as described above also, it is possible to obtain functions and effects similar to those of the injection needle assembly 1 according to the above-described first embodiment.

Note that the present invention is not limited to the embodiments described above and illustrated in figures, and various modifications can be possible within the scope not departing from the gist of the invention described in claims. For example, the outer housing unit may be configured to slide so as to cover the outside in the radial direction of the guide part.

Explanation of Reference Numerals

1, 20, 30: injection needle assembly, 2: needle tube, 2a: blade face, 3: hub, 4: adjustment unit, 4b: needle protruding surface, 5: adhesive agent, 6, 21, 31: stabilization unit, 6a, 21a, 31a: fixing part, 6b, 21b, 31b: contact part, 6c: end face, 6d: sliding groove, 6e, 31c, 31d: engaging groove, 7, 23: outer housing unit (housing unit), 7a, 23a, 38a: covering part, 7b,38b: leg part, 7c, 38c: engaging claw, 8, 38: internal housing unit (housing unit), 8c: insertion hole, 9, 22, 32: guide part, 9a, 22a, 32a: contact surface, 11: syringe, 12: connection pin, 22b: opening part, B: bevel length, L: protrusion length, S: distance from the circumferential edge of the needle protruding surface to the circumferential surface of the needle tube, T: distance from the internal wall surface of the stabilization unit to the outer circumferential surface of the adjustment unit, x: guide part length, y: guide part height, d: internal diameter

**Claims**

1. An injection needle assembly comprising:

   a needle tube having a needle tip capable of being stuck into a living body;
   a hub configured to hold the needle tube;
   a stabilization unit arranged so as to cover the circumference of the needle tube in a condition that the needle tip of the needle tube protrudes, the stabilization unit having an end face that contacts a skin when causing the needle tube to be stuck into the living body; and
   a housing unit movable along an axial direction of the needle tube from a first position where the needle tip of the needle tube is exposed to a second position where the needle tip of the needle tube is housed.

2. The injection needle assembly according to claim 1, wherein the housing unit is arranged so as to cover the circumference of the stabilization unit.

3. The injection needle assembly according to claim 1, wherein the housing unit is arranged so as to cover the circumference of the needle tube between the stabilization unit and the needle tube.

4. The injection needle assembly according to claim 1, wherein the housing unit includes an outer housing unit arranged so as to cover the circumference of the stabilization unit and an inner housing unit arranged so as to cover the circumference of the needle tube between the stabilization unit and the needle tube, and the outer housing unit and the inner housing unit are integrally formed.

5. The injection needle assembly according to any one of claims 1 through 4, wherein the housing unit slides against the stabilization unit.

6. The injection needle assembly according to any one of claims 1 through 5, wherein a fixing mechanism configured to fix the housing unit at the second position and prevent movement of the housing unit to the first position is provided in the stabilization unit and the housing unit.

7. The injection needle assembly according to any one of claims 1 through 6, wherein a guide part is provided in the stabilization unit or the housing unit, the guide part ensuring that a pressing force of the needle tube and the stabilization unit to the living body is at a predetermined value or above by being caused to contact the skin when causing the needle tube to be stuck into the living body.

8. The injection needle assembly according to claims 1 through 7, wherein an adjustment unit having a needle protruding surface from which the needle tip of the needle tube protrudes is provided around the needle tube.

9. A drug injection device comprising:

a needle tube having a needle tip capable of being stuck into a living body;

a hub configured to hold the needle tube;

a syringe configured to be connected with the hub;

a stabilization unit arranged so as to cover the circumference of the needle tube in a condition that the needle tip of the needle tube protrudes, the stabilization unit having an end face that contacts a skin when causing the needle tube to be stuck into the living body; and

a housing unit movable along an axial direction of the needle tube from a first position where the needle tip of the needle tube is exposed to a second position where the needle tip of the needle tube is housed.

# FIG. 1

# FIG. 2

# FIG. 3

# FIG. 4

# FIG. 5

## FIG. 6

# FIG. 7

# FIG. 8

# FIG. 9

# FIG. 10

# FIG. 11

# FIG. 12

## INTERNATIONAL SEARCH REPORT

| | International application No. |
|---|---|
| | PCT/JP2010/053681 |

**A. CLASSIFICATION OF SUBJECT MATTER**
*A61M5/32*(2006.01)i

According to International Patent Classification (IPC) or to both national classification and IPC

**B. FIELDS SEARCHED**

Minimum documentation searched (classification system followed by classification symbols)
A61M5/32

Documentation searched other than minimum documentation to the extent that such documents are included in the fields searched
Jitsuyo Shinan Koho          1922-1996     Jitsuyo Shinan Toroku Koho     1996-2010
Kokai Jitsuyo Shinan Koho     1971-2010     Toroku Jitsuyo Shinan Koho     1994-2010

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2004-526521 A  (Becton, Dickinson and Co.),<br>02 September 2004 (02.09.2004),<br>paragraphs [0024] to [0025]; fig. 8A, 8B<br>& WO 2002/083213 A1 | 1,2,5-7,9<br>3,4,8 |
| Y | JP 2006-516461 A  (Becton, Dickinson and Co.),<br>06 July 2006 (06.07.2006),<br>paragraph [0013]; fig. 4 to 6<br>& US 2006/0276756 A1     & WO 2004/069302 A2 | 3,4,8 |

☐ Further documents are listed in the continuation of Box C.     ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 March, 2010 (25.03.10) | 06 April, 2010 (06.04.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2010/053681 |

---

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III      Observations where unity of invention is lacking (Continuation of item 3 of first sheet)**

This International Searching Authority found multiple inventions in this international application, as follows:
    The search revealed that the invention in claim 1 is not novel, since the invention is disclosed in JP 2004-526521 A.
    Therefore, the technical feature of the invention in claim 1 cannot be considered to be a special technical feature in the meaning of the second sentence of PCT Rule 13.2.
    Consequently, it is not recognized that there is one or more same or corresponding special technical feature among the inventions in claims 1 – 9.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001137343 A

**Non-patent literature cited in the description**

- **R. T. Kenney et al.** *New England Journal of Medicine,* 2004, vol. 351, 2295-2301